# EUROPEAN PATENT APPLICATION

(11) **EP 4 501 872 A1**
(43) Date of publication of application: **05.02.2025**
(21) Application number: 22935757.9
(22) Date of filing: 22.12.2022
(51) Int. Cl.: C03C 3/095, A61K 6/25, A61K 6/77, A61K 6/836, A61K 6/84, A61K 6/842, C03C 3/062, C03C 3/076

(54) **GLASS COMPOSITION**

(30) Priority: 31.03.2022 JP 2022061126
(71) Applicant: GC Corporation, Sunto-gun, Shizuoka 410-1307 (JP)
(72) Inventor: MIYAKE, Takahiro, Tokyo 174-8585 (JP); FUNAYAMA, Naoya, Tokyo 174-8585 (JP); SHIMADA, Yusuke, Tokyo 174-8585 (JP)
(74) Representative: Müller-Boré & Partner Patentanwälte PartG mbB
(86) International application number: PCT/JP2022/047407
(87) International publication number: WO 2023/188631

(57) **Abstract**

A glass composition includes gadolinium (Gd), cesium (Cs), or both; and silicon (Si). In the glass composition, in terms of respective oxides thereof, a total content of a gadolinium oxide and a cesium oxide is 1% by mass or more, and a content of a silicon oxide is 30% by mass or more.

## Description

### TECHNICAL FIELD

The present invention relates to a glass composition.

### BACKGROUND ART

Barium glass is known as radiopacity glass (see, for example, PTL 1). Radiopacity means X-ray opacity. The radiopacity becomes better as the X-ray opacity becomes higher, i.e., X-ray transmittance becomes lower.

### CITATION LIST

### PATENT LITERATURE

PTL 1: Japanese Patent No. 6346086

### SUMMARY OF THE INVENTION

### TECHNICAL PROBLEM

In one aspect, the present invention provides a glass composition that is excellent in radiopacity and aesthetics.

### SOLUTION TO THE PROBLEM

A glass composition according to one aspect of the present invention includes: gadolinium (Gd), cesium (Cs), or both; and silicon (Si). In the glass composition, in terms of respective oxides thereof, a total content of a gadolinium oxide and a cesium oxide is 1% by mass or more, and a content of a silicon oxide is 30% by mass or more.

### ADVANTAGEOUS EFFECTS OF THE INVENTION

According to one aspect of the present invention, it is possible to provide a glass composition that is excellent in radiopacity and aesthetics.

### DETAILED DESCRIPTION OF THE INVENTION

Hereinafter, embodiments of the present invention will be described. In the specification, "through" indicating a numerical range means that numerical values described before and after that are included as the lower limit and the upper limit.

The glass composition of the present embodiment includes: gadolinium (Gd), cesium (Cs), or both; and silicon (Si). The glass composition may include either gadolinium or cesium, but preferably includes both of gadolinium and cesium.

Gadolinium is a rare earth element in which the 4f orbital capable of containing up to 14 electrons is occupied by seven electrons, i.e., half the number of electrons that can occupy the orbital, and all occupied electrons are unpaired electrons. In this manner, the 4f orbital is occupied by seven unpaired electrons, and thus gadolinium has the largest magnetic moment among the 4f elements.

Cesium (atomic number: 55) has the second largest atomic number after barium (atomic number: 56). Thus, cesium can exhibit high radiopacity. Also, cesium is an alkali metal element, and can develop mixed alkali ion effects when cesium is mixed with other alkali metal elements (e.g., sodium), thereby increasing water resistance and chemical resistance.

By including gadolinium, cesium, or both, the glass composition can reduce the content of barium (Ba) while suppressing reduction in radiopacity. Also, the glass composition can be substantially free of barium. The radiopacity is X-ray opacity. The radiopacity becomes better as the X-ray opacity becomes higher, i.e., X-ray transmittance becomes lower.

The radiopacity of glass compositions is determined by the test method for radiopacity of dental materials in accordance with ISO 13116:2014. Specifically, first, the optical density or gray value of a test piece (a plate of a glass composition) having a thickness of Ts (in mm) is measured. Next, a thickness Ta (in mm) of an aluminum plate exhibiting the same optical density or gray value as the test piece having the thickness of Ts is determined. Then, the ratio of Ta to Ts (in %) is used as a value of the radiopacity.

As the ratio of Ta to Ts is higher, the radiopacity (X-ray opacity) is better. The ratio of Ta to Ts is preferably 200% or higher, more preferably 400% or higher, further preferably 600% or higher, and especially preferably 800% or higher. The ratio of Ta to Ts is preferably 1,200% or lower.

The glass composition includes gadolinium, cesium, or both as described above, and thus can reduce the content of barium while suppressing reduction in radiopacity. Thereby, the refractive index of the glass composition can be adjusted to be 1.45 or higher and lower than 1.56. In the present specification, the refractive index means a refractive index in the D line (a light beam having a wavelength of 589 nm).

When the refractive index of the glass composition is 1.45 or higher and lower than 1.56, the difference in refractive index between the below-described polymerizable monomer and the glass composition can be reduced. This can increase transparency of a dental polymerizable composition as described below, and thus can improve aesthetics thereof. The refractive index of the polymerizable monomer is approximately 1.50 or higher and 1.52 or lower.

The refractive index of the glass composition is preferably 1.45 or higher and lower than 1.56 as described above. The refractive index of the glass composition is more preferably 1.50 or higher, and further preferably 1.52 or higher. The refractive index of the glass composition is more preferably 1.54 or lower.

In the glass composition, in terms of respective oxides thereof, the total content of a gadolinium oxide (Gd₂O₃) and a cesium oxide (Cs₂O) is preferably 1% by mass or more. When the total content of the gadolinium oxide and the cesium oxide is 1% by mass or more, the obtained glass composition can be excellent in radiopacity and aesthetics.

In the glass composition, the total content of the gadolinium oxide and the cesium oxide is more preferably 5% by mass or more, and more preferably 9% by mass or more. The total content of the gadolinium oxide and the cesium oxide is more preferably 50% by mass or less, and more preferably 40% by mass or less.

In the glass composition, in terms of an oxide, the content of the gadolinium oxide is preferably from 0% by mass through 40% by mass, more preferably from 12% by mass through 30% by mass, and further preferably from 15% by mass through 25% by mass. When the content of the gadolinium oxide is 40% by mass or lower, it is possible to suppress coloring of the dental polymerizable composition after curing. The content of the gadolinium oxide is preferably 1% by mass or more.

In the glass composition, in terms of an oxide, the content of the cesium oxide is preferably from 0% by mass through 20% by mass, more preferably from 5% by mass through 17% by mass, and further preferably from 8% by mass through 15% by mass. The content of the cesium oxide is preferably from 1% by mass or more.

The glass composition may be substantially free of barium, but may include barium. By including barium, the contents of gadolinium and cesium in the glass composition can be reduced. In the glass composition, in terms of an oxide, the content of barium oxide (BaO) in the glass composition is preferably 0% by mass or more and less than 30% by mass. When the content of the barium oxide is less than 30% by mass, the refractive index of the glass composition can be adjusted to be lower than 1.56. The content of the barium oxide is more preferably from 8% by mass through 25% by mass, and further preferably from 13% by mass through 22% by mass.

In the glass composition, in terms of an oxide, the content of silicon oxide (SiO₂) is preferably from 30% by mass through 80% by mass, more preferably from 35% by mass through 70% by mass, and further preferably from 45% by mass through 65% by mass. When the content of the silicon oxide is 30% by mass or more, the viscosity of the dental polymerizable composition can be readily adjusted, and the mechanical strength thereof can be increased. When the content of the silicon oxide is 80% by mass or less, reduction in radiopacity can be suppressed.

The glass composition may include elements other than gadolinium, cesium, barium, and silicon. Examples of the other elements include aluminum (Al), boron (B), sodium (Na), and fluorine (F).

In the glass composition, in terms of an oxide, the content of aluminum oxide (Al₂O₃) is from 0% by mass through 20% by mass, more preferably from 3% by mass through 15% by mass, and further preferably from 5% by mass through 10% by mass. When the content of the aluminum oxide is 3% by mass or more, it is possible to increase chemical durability and mechanical strength. When the content of the aluminum oxide is 20% by mass or less, it is possible to suppress devitrification and reduction in acid resistance.

In the glass composition, in terms of an oxide, the content of boron oxide (B₂O₃) is from 0% by mass through 14% by mass, more preferably from 3% by mass through 12% by mass, and further preferably from 5% by mass through 8% by mass. When the content of the boron oxide is 3% by mass or more, it is possible to suppress crystallization of the resulting glass. When the content of the boron oxide is 14% by mass or less, it is possible to suppress reduction in chemical durability and unevenness in refractive index.

In the glass composition, in terms of an oxide, the content of sodium oxide (Na₂O) is preferably from 0% by mass through 20% by mass, more preferably from 3% by mass through 15% by mass, and further preferably from 5% by mass through 10% by mass. When the content of the sodium oxide is 20% by mass or less, it is possible to suppress reduction in chemical durability and reduction in water resistance.

In the glass composition, the content of fluorine (F) is preferably from 0% by mass through 5% by mass, more preferably from 0% by mass through 3% by mass, and more preferably from 0% by mass through 1% by mass. When the content of fluorine is 5% by mass or less, the viscosity of the dental polymerizable composition is readily adjusted.

The glass composition is in a powder form or in a plate form. The glass composition in a powder form is glass powder, and the glass composition in a plate form is a glass plate. When the glass composition is used as a part of the dental polymerizable composition, the glass composition is preferably in a powder form. A method of grinding a glass plate into glass powder may be a typical method, and may be a dry or wet method. A planetary mill, a vibration mill, a ball mill, or a bead mill is used.

When the glass composition is in a powder form, the average particle diameter thereof is preferably from 80 nm through 1,000 nm, more preferably from 100 nm through 900 nm, and further preferably from 150 nm through 800 nm. In the present specification, the average particle diameter refers to a volume-basis median diameter D50 as measured through dynamic light scattering.

The glass composition can also be used as glass for blocking radioactive rays. In this case, the glass composition may be used in a plate form or may be used in a powder form and mixed with acrylic. In either case, the amount of lead to be used can be reduced by using the glass composition of the present embodiment instead of lead glass.

Next, details of the dental polymerizable composition will be described. The dental polymerizable composition includes the glass composition. The content of the glass composition in the dental polymerizable composition is preferably 40% by mass or more and 90% by mass or less, more preferably 50% by mass or more and 80% by mass or less, and further preferably 60% by mass or more and 70% by mass or less.

When the content of the glass composition in the dental polymerizable composition is 40% by mass or more, it is possible to impart high radiopacity to the dental polymerizable composition, and increase the mechanical strength thereof. When the content of the glass composition in the dental polymerizable composition is 90% by mass or less, the viscosity of the dental polymerizable composition is low, and the operability of the dental polymerizable composition can be improved.

The dental polymerizable composition includes a (meth)acrylate compound in addition to the glass composition as described above. In the present specification, the polymerizable composition refers to a composition having the function of undergoing polymerization. The (meth)acrylate compound refers to a monomer, oligomer, or prepolymer having one or more (meth)acryloyloxy groups of (meth)acrylate. In the present specification, the (meth)acrylate refers to either or both of acrylate and methacrylate.

No particular limitation is imposed on the (meth)acrylate, which may be, for example, an acid group-including (meth)acrylate, an acid group-free (meth)acrylate, or both. The acid group-including (meth)acrylate may include a plurality of acid groups. Also, instead of the acid group-including (meth)acrylate, an acid chloride, alkali metal salt, and amine salt of the acid group-including (meth)acrylate may be used.

Examples of the acid group-including (meth)acrylate include phosphoric acid group-including (meth)acrylates, pyrophosphoric acid group-including (meth)acrylates, thiophosphoric acid group-including (meth)acrylates, carboxyl group-including (meth)acrylates, sulfonic acid group-including (meth)acrylates, and phosphonic acid group-including (meth)acrylates, and the like.

Examples of the phosphoric acid group-including (meth)acrylates include 2-(meth)acryloyloxyethyl dihydrogen phosphate, bis[2-(meth)acryloyloxyethyl] hydrogen phosphate, 2-(meth)acryloyloxyethylphenyl hydrogen phosphate, 6-(meth)acryloyloxyhexyl dihydrogen phosphate, 6-(meth)acryloyloxyhexylphenyl hydrogen phosphate, 10-(meth)acryloyloxydecyl dihydrogen phosphate, 1,3-di(meth)acryloylpropan-2-dihydrogen phosphate, 1,3-di(meth)acryloylpropan-2-phenylhydrogen phosphate, bis[5-{2-(meth)acryloyloxyethoxycarbonyl}heptyl] hydrogen phosphate, and the like. Of these, 10-(meth)acryloyloxydecyl dihydrogen phosphate is preferable in terms of increasing adhesiveness of the dental polymerizable composition.

Examples of the pyrophosphoric acid group-including (meth)acrylates include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, bis[10-(meth)acryloyloxydecyl] pyrophosphate, and the like.

Examples of the thiophosphoric acid group-including (meth)acrylates include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 13-(meth)acryloyloxytridecyl dihydrogen thiophosphate, 14-(meth)acryloyloxytetradecyl dihydrogen thiophosphate, 15-(meth)acryloyloxypentadecyl dihydrogen thiophosphate, 16-(meth)acryloyloxyhexadecyl dihydrogen thiophosphate, 17-(meth)acryloyloxyheptadecyl dihydrogen thiophosphate, 18-(meth)acryloyloxyoctadecyl dihydrogen thiophosphate, 19-(meth)acryloyloxynonadecyl dihydrogen thiophosphate, 20-(meth)acryloyloxyicosyl dihydrogen thiophosphate, and the like.

Examples of the carboxyl group-including (meth)acrylates include 2-methacryloyloxyethyl succinate, 4-(meth)acryloyloxyethyl trimellitate, 4-(meth)acryloyloxyethyl trimellitic anhydride, 4-(meth)acryloyloxydecyl trimellitate, 4-(meth)acryloyloxydecyl trimellitic anhydride, 11-(meth)acryloyloxy-1,1-undecanedicarboxylate, 1,4-di(meth)acryloyloxy pyromellitate, 2-(meth)acryloyloxyethyl maleate, 2-(meth)acryloyloxyethyl phthalate, 2-(meth)acryloyloxyethyl hexahydrophthalate, and the like. Of these, 2-methacryloyloxyethyl succinate is preferable in terms of increasing adhesiveness of the dental polymerizable composition.

Examples of the sulfonic acid group-including (meth)acrylates include 2-(meth)acrylamide-2-methylpropanesulfonate, styrenesulfonate, 2-sulfoethyl (meth)acrylate, and the like.

Examples of the phosphonic acid group-including (meth)acrylates include 2-(meth)acryloyloxyethyl phenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and the like.

These acid group-including (meth)acrylates may be used alone or in combination.

Of the acid group-including (meth)acrylates as described above, the phosphoric acid group- or carboxyl group-including (meth)acrylates are preferable in terms of solubility of the smear layer and decalcification on the tooth surface exhibited by the dental polymerizable composition, and especially in terms of adhesiveness to the enamel.

No particular limitation is imposed on the content of the acid group-including (meth)acrylate in the dental polymerizable composition. The content of the acid group-including (meth)acrylate in the dental polymerizable composition can be, for example, 0.1% by mass or more and 20% by mass or less, preferably 0.1% by mass or more and 15% by mass or less, and more preferably 0.1% by mass or more and 10% by mass or less. When the content of the acid group-including (meth)acrylate in the dental polymerizable composition is 0.1% by mass or more, adhesiveness of the dental polymerizable composition to the tooth substance is further increased. When the content of the acid group-including (meth)acrylate in the dental polymerizable composition is 20% by mass or less, curability of the dental polymerizable composition is increased.

Examples of the acid group-free (meth)acrylate include methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-methylhexyl (meth)acrylate, 2-ethylhexyl (meth)acrylate, benzyl (meth)acrylate, 2-hydroxy-1,3-di(meth)acryloyloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, bisphenol A diglycidyl (meth)acrylate, di-2-(meth)acryloyloxyethyl-2,2,4-trimethylhexamethylene dicarbamate, 1,3,5-tris[1,3-bis{(meth)acryloyloxy}-2-propoxycarbonylaminohexane]-1,3,5-(1H,3H,5H)triazine-2,4,6-trione, 2,2-bis[4-(3-(meth)acryloyloxy-2-hydroxypropyl)phenyl]propane, N,N'-(2,2,4-trimethylhexamethylene)bis[2-(aminocarboxy)propane-1,3-diol]tetramethacrylate, and the like.

These acid group-free (meth)acrylates may be used alone or in combination.

Of the acid group-free (meth)acrylates as described above, di-2-(meth)acryloyloxyethyl-2,2,4-trimethylhexamethylene dicarbamate and 2-hydroxy-1,3-di(meth)acryloyloxypropane are preferable in terms of increasing the mechanical strength of the cured product of the dental polymerizable composition.

The content of the acid group-free (meth)acrylate in the dental polymerizable composition may be 10% by mass or more and 95% by mass or less, preferably 13% by mass or more and 90% by mass or less, and more preferably 15% by mass or more and 80% by mass or less.

When the content of the acid group-free (meth)acrylate in the dental polymerizable composition is 10% by mass or more, the operability of the dental polymerizable composition is improved. When the content of the acid group-free (meth)acrylate in the dental polymerizable composition is 95% by mass or less, it is possible to increase the mechanical strength of the crude product of the dental polymerizable composition.

The dental polymerizable composition preferably includes an inorganic filler in addition to the glass composition as described above. No particular limitation is imposed on the component of the inorganic filler. Examples of the component of the inorganic filler include colloidal silica, surface-hydrophobized particulate silica, aluminum oxide, fluoroaluminosilicate glass, barium glass, and the like. These inorganic fillers may be used alone or in combination. In the following description, the inorganic filler means an inorganic component other than the glass composition.

The content of the inorganic filler in the dental polymerizable composition is, for example, preferably 0.01% by mass or more and 30% by mass or less, more preferably 0.05% by mass or more and 20% by mass or less, and further preferably 0.1% by mass or more and 10% by mass or less. When the content of the inorganic filler in the dental polymerizable composition is 0.01% by mass or more, it is possible to increase the viscosity of the dental polymerizable composition, and thus improve the operability of the dental polymerizable composition. When the content of the inorganic filler in the dental polymerizable composition is 30% by mass or less, the viscosity of the dental polymerizable composition does not become too high, and thus high operability of the dental polymerizable composition can be maintained.

The dental polymerizable composition may include other components, e.g., at least one selected from chemical polymerization initiators, photopolymerization initiators, and polymerization inhibitors.

No particular limitation is imposed on the chemical polymerization initiator, which may be a thiourea derivative, a vanadium compound, a tertiary amine, or an organic peroxide. Of these chemical polymerization initiators, a thiourea derivative, a vanadium compound, and a tertiary amine function as a reducing agent, and an organic peroxide functions as an oxidizing agent.

No particular limitation is imposed on the thiourea derivative. Examples of the thiourea derivative include ethylene thiourea, N-methylthiourea, N-ethylthiourea, N-propylthiourea, N-butylthiourea, N-laurylthiourea, N-phenylthiourea, N-cyclohexylthiourea, N,N-dimethylthiourea, N,N-diethylthiourea, N,N-dipropylthiourea, N,N-dibutylthiourea, N,N-dilaurylthiourea, N,N-diphenylthiourea, N,N-dicyclohexylthiourea, trimethylthiourea, tetramethylthiourea, N-acetylthiourea, N-benzoylthiourea, 1-allyl-3-(2-hydroxyethyl)-2-thiourea, 1-(2-tetrahydrofurfuryl)-2-thiourea, N-tert-butyl-N'-isopropylthiourea, 2-pyridylthiourea, and the like. These thiourea derivatives may be used alone or in combination. Of these, N-benzoylthiourea is preferable in terms of increasing the curability of the dental polymerizable composition.

No particular limitation is imposed on the content of the thiourea derivative in the dental polymerizable composition. The content of the thiourea derivative in the dental polymerizable composition is preferably 0.1% by mass or more and 5% by mass or less, more preferably 0.1% by mass or more and 3% by mass or less, and further preferably 0.1% by mass or more and 1% by mass or less. When the content of the thiourea derivative in the dental polymerizable composition is 0.1% by mass or more, the curability of the dental polymerizable composition is further increased. When the content of the thiourea derivative in the dental polymerizable composition is 5% by mass or less, the solubility of the thiourea derivative in the (meth)acrylate in the dental polymerizable composition is increased.

No particular limitation is imposed on the vanadium compound. Examples of the vanadium compound include oxovanadium oxalate, vanadyl acetylacetonate, vanadium acetylacetonate, vanadyl stearate, vanadium naphthenate, vanadium benzoyl acetonate, and the like. These may be used alone or in combination. Of these, vanadyl acetylacetonate is preferable in terms of the curability of the dental polymerizable composition.

No particular limitation is imposed on the content of the vanadium compound in the dental polymerizable composition. The content of the vanadium compound in the dental polymerizable composition is preferably 0.001% by mass or more and 5% by mass or less, more preferably 0.0015% by mass or more and 1% by mass or less, and further preferably 0.002% by mass or more and 0.1% by mass or less. When the content of the vanadium compound in the dental polymerizable composition is 0.001% by mass or more, the curability of the dental polymerizable composition is further increased. When the content of the vanadium compound in the dental polymerizable composition is 5% by mass or less, the storage stability of the dental polymerizable composition is further improved.

No particular limitation is imposed on the tertiary amine. Examples of the tertiary amine include tertiary aliphatic amines and tertiary aromatic amines. Examples of the tertiary aliphatic amines include N,N-dimethylaminoethyl methacrylate, triethanolamine, and the like. Examples of the tertiary aromatic amines include alkyl p-dialkylaminobenzoate, 7-dimethylamino-4-methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

Of these, the tertiary amine is preferably a tertiary aromatic amine, and more preferably alkyl p-dialkylaminobenzoate. Examples of the alkyl p-dialkylaminobenzoate include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like.

These tertiary amines may be used alone or in combination.

Examples of the organic peroxide include benzoyl peroxide, cumene hydroperoxide, t-butyl hydroperoxide, t-amyl hydroperoxide, 1,1,3,3-tetramethylbutyl hydroperoxide, 2,5-dimethyl-2,5-di(hydroperoxy)hexane, p-diisopropylbenzene monohydroperoxide, p-methane hydroperoxide, pinane hydroperoxide, and the like. These organic peroxides may be used alone or in combination. Of these, cumene hydroperoxide is preferable in terms of the curability of the dental polymerizable composition.

No particular limitation is imposed on the content of the organic peroxide in the dental polymerizable composition. The content of the organic peroxide in the dental polymerizable composition is preferably 0.01% by mass or more and 10% by mass or less, more preferably 0.05% by mass or more and 5% by mass or less, and further preferably 0.1% by mass or more and 3% by mass or less. When the content of the organic peroxide in the dental polymerizable composition is 0.01% by mass or more, the curability of the dental polymerizable composition is further increased. When the content of the organic peroxide in the dental polymerizable composition is 10% by mass or less, the window during which the dental polymerizable composition can be used is further increased.

No particular limitation is imposed on the photopolymerization initiator. Examples of the photopolymerization initiator include camphorquinone, phenylbis(2,4,6-trimethylbenzoyl)phosphine oxide, 2,4,6-trimethylbenzoyl diphenylphosphine oxide, benzyl ketal, diacetyl ketal, benzyl dimethyl ketal, benzyl diethyl ketal, benzyl bis (2-methoxyethyl) ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl)ketone, 4,4'-bis(diethylamino)benzophenone, and the like. These photopolymerization initiators may be used alone or in combination. Of these, camphorquinone and phenylbis(2,4,6-trimethylbenzoyl)phosphine oxide are preferable in terms of increasing the curability of the dental polymerizable composition.

No particular limitation is imposed on the content of the photopolymerization initiator in the dental polymerizable composition. The content of the photopolymerization initiator in the dental polymerizable composition is preferably 0.001% by mass or more and 1% by mass or less, more preferably 0.005% by mass or more and 0.5% by mass or less, and further preferably 0.01% by mass or more and 0.3% by mass or less. When the content of the photopolymerization initiator in the dental polymerizable composition is 0.001% by mass or more, the curability of the dental polymerizable composition is further increased. When the content of the photopolymerization initiator in the dental polymerizable composition is 0.3% by mass or less, the storage stability of the dental polymerizable composition is further improved.

Examples of the polymerization inhibitor include dibutylhydroxytoluene (2,6-di-tert-butyl-p-cresol), 6-tert-butyl-2,4-xylenol, and the like. These polymerization inhibitors may be used alone or in combination. Of these, dibutylhydroxytoluene is preferable in terms of increasing the curability of the dental polymerizable composition.

No particular limitation is imposed on the content of the polymerization inhibitor in the dental polymerizable composition. The content of the polymerization inhibitor in the dental polymerizable composition is preferably 0.001% by mass or more and 5% by mass or less, more preferably 0.005% by mass or more and 1% by mass or less, and further preferably 0.01% by mass or more and 0.1% by mass or less. When the content of the polymerization inhibitor in the dental polymerizable composition is 0.001% by mass or more and 5% by mass or less, the storage stability of the dental polymerizable composition is improved.

No particular limitation is imposed on the formulation of the dental polymerizable composition. The formulation of the dental polymerizable composition may be, for example, a two-part formulation including a first agent and a second agent, or a one-part formulation including only a first agent. In the case of the two-part formulation, the first agent and the second agent may be kneaded and used. The mass ratio of the first agent and the second agent is preferably from 10:1 through 1:10 and more preferably from 1:5 through 5:1.

In the case of the two-part formulation, both of the first agent and the second agent preferably include the glass composition as described above. This can enhance radiopacity. Both of the first agent and the second agent preferably include the glass composition as described above and the (meth)acrylate compound as described above, and more preferably include the inorganic filler as described above. Both of the first agent and the second agent may further include other components, such as the chemical polymerization initiators as described above and the like.

In the case of the one-part formulation, the first agent includes the glass composition as described above. This can enhance radiopacity. the first agent includes the glass composition as described above and the (meth)acrylate compound as described above, and preferably includes the inorganic filler as described above. The first agent may further include other components, such as the chemical polymerization initiator as described above and the like.

No particular limitation is imposed on the use of the dental polymerizable composition. The dental polymerizable composition may be used, for example, in a variety of dental materials. Examples of the dental materials include dental cements, dental adhesives, dental temporary sealants, dental primers, dental coatings, dental composite resins, dental hard resins, dental cutting resins, dental temporary restoration materials, dental fillers, dentifrices, and the like. Of these, the dental polymerizable composition is preferably used for dental cements.

### EXAMPLES

Experimental data will be described below. In Examples 1 to 7 and Comparative Example 1, glass plates having the chemical compositions shown in Table 5 were produced, and measured for radiopacity of the glass plates and refractive index of glass powder thereof. The chemical compositions of the glass plates were measured through X-ray fluorescence spectrometry. The radiopacity of the glass plates was measured as described above. The thickness Ts of the glass plate, a test piece, was 1 mm.

The refractive index of the glass powder was measured in accordance with the following procedure. First, the glass plate was ground to produce the glass powder having an average particle diameter of 1,000 nm. Then, liquid having a specific refractive index and the produced glass powder were mixed at 1:1 (mass ratio) and packed in a transparent tube. Next, the transparent tube was held up to a white LED, and the color of light transmitting through the transparent tube was visually observed. Then, the refractive index of the liquid, when the light transmitting through the transparent tube turned green, was used as the refractive index of the glass powder. When the refractive index of the liquid is lower than that of the glass powder, the color of light transmitting through the transparent tube turns blue. When the refractive index of the liquid is higher than that of the glass powder, the color of light transmitting through the transparent tube turns red or yellow.

When the refractive index of the glass powder is from 1.4600 through 1.5400, Liquid 1 or Liquid 2 as described in Table 1, or a mixture thereof was used as the liquid. Both of Liquid 1 and Liquid 2 are typical as dental materials.

**[Table 1]**

| Refractive index [-] | 1.4600 | 1.4604 | 1.4608 | 1.4612 | 1.4616 | ··· | 1.5400 |
|---|---|---|---|---|---|---|---|
| Liquid 1 [% by mass] | 100.0 | 99.5 | 99.0 | 98.5 | 98.0 | ... | 0.0 |
| Liquid 2 [% by mass] | 0.0 | 0.5 | 1.0 | 1.5 | 2.0 | ··· | 100.0 |

| | | | | | | | |
|---|---|---|---|---|---|---|---|
| Liquid 1: Triethylene Glycol Dimethacylate Liquid 2: Ethoxylated Bisphenol A Dimethacrylate | | | | | | | |

The mixing ratio (mass ratio) of Liquid 1 and Liquid 2 is adjusted in 0.5% increments.

When the refractive index of the glass powder was from 1.5400 through 1.6600, Liquid 2 or Liquid 3 as described in Table 2, or a mixture thereof was used as the liquid. Both of Liquid 2 and Liquid 3 are typical as dental materials.

**[Table 2]**

| Refractive index [-] | 1.5400 | 1.5406 | 1.5412 | 1.5418 | 1.5424 | ... | 1.6600 |
|---|---|---|---|---|---|---|---|
| Liquid 2 [% by mass] | 100.0 | 99.5 | 99.0 | 98.5 | 98.0 | ... | 0.0 |
| Liquid 3 [% by mass] | 0.0 | 0.5 | 1.0 | 1.5 | 2.0 | ... | 100.0 |

| | | | | | | | |
|---|---|---|---|---|---|---|---|
| Liquid 2: Ethoxylated Bisphenol A Dimethacrylate Liquid 3: 1-Bromonaphthalene | | | | | | | |

The mixing ratio (mass ratio) of Liquid 2 and Liquid 3 is adjusted in 0.5% increments.

In Examples 1 to 7 and Comparative Example 1, the refractive index of the glass powder was from 1.4600 through 1.6600. However, when the refractive index of the glass powder is from 1.4500 through 1.4600, Liquid 1 or Liquid 4 as described in Table 3, or a mixture thereof may be used as the liquid. Both of Liquid 1 and Liquid 4 are typical as dental materials.

**[Table 3]**

| Refractive index [-] | 1.4600 | 1.4595 | 1.4590 | 1.4585 | 1.4580 | ··· | 1.4500 |
|---|---|---|---|---|---|---|---|
| Liquid 1 [% by mass] | 100.0 | 95.0 | 90.0 | 85.0 | 80.0 | ... | 0.0 |
| Liquid 4 [% by mass] | 0.0 | 5.0 | 10.0 | 15.0 | 20.0 | ··· | 100.0 |

| | | | | | | | |
|---|---|---|---|---|---|---|---|
| Liquid 1: Triethylene Glycol Dimethacylate Liquid 4: 2-Hydroxyethyl Methacrylate | | | | | | | |

The mixing ratio (mass ratio) of Liquid 1 and Liquid 4 is adjusted in 5.0% increments.

When the refractive index of the glass powder is from 1.3300 through 1.4500, Liquid 4 or Liquid 5 as described in Table 4, or a mixture thereof may be used as the liquid. Both of Liquid 4 and Liquid 5 are typical as dental materials.

**[Table 4]**

| Refractive index [-] | 1.4500 | 1.4494 | 1.4488 | 1.4482 | 1.4476 | ... | 1.3300 |
|---|---|---|---|---|---|---|---|
| Liquid 4 [% by mass] | 100.0 | 99.5 | 99.0 | 98.5 | 98.0 | ... | 0.0 |
| Liquid 5 [% by mass] | 0.0 | 0.5 | 1.0 | 1.5 | 2.0 | ··· | 100.0 |

| | | | | | | | |
|---|---|---|---|---|---|---|---|
| Liquid 4: 2-Hydroxyethyl Methacrylate Liquid 5: Distilled Water | | | | | | | |

The mixing ratio (mass ratio) of Liquid 4 and Liquid 5 is adjusted in 0.5% increments.

The refractive index of the glass powder is measured as described above. The refractive index of the glass plate can be measured by the V-block method.

The physical properties of the glass plates produced in Examples 1 to 7 and Comparative Example 1 are shown in Table 5.

**[Table 5]**

| | Example 1 | Example 2 | Example 3 | Example 4 | Example 5 | Example 6 | Example 7 | Comparative Example 1 |
|---|---|---|---|---|---|---|---|---|
| Gd₂O₃ [% by mass] | 14.2 | 39.2 | | 12.4 | 19.5 | 20.0 | 7.3 | |
| Cs₂O [% by mass] | | | 9.8 | 9.4 | 9.4 | 10.5 | | |
| SiO₂ [% by mass] | 37.5 | 48.4 | 74.5 | 69.3 | 63.0 | 61.0 | 41.3 | 46.6 |
| B₂O₃ [% by mass] | 13.3 | | | | | | 12.2 | 13.2 |
| F [% by mass] | 0.9 | | | | | | 1.2 | 0.9 |
| Na₂O [% by mass] | 0.2 | 10.9 | 9.7 | 8.5 | 7.7 | 8.1 | 0.1 | 0.2 |
| Al₂O₃ [% by mass] | 9.1 | | 0.3 | 0.4 | 0.4 | 0.4 | 10.3 | 9.1 |
| BaO [% by mass] | 24.8 | 1.5 | 5.7 | | | | 27.6 | 300 |
| Gd₂O₃+Cs₂O [% by mass] | 14.2 | 39.2 | 9.8 | 21.8 | 28.9 | 30.5 | 7.3 | 0.0 |
| Refractive index [-] | 1.5502 | 1.5300 | 1.4900 | 1.5050 | 1.5250 | 1.5350 | 1.5400 | 1.5600 |
| Radiopacity (Ta/Ts) [%] | 1001 | 406 | 258 | 405 | 570 | 595 | 823 | 750 |

As shown in Table 5, according to Examples 1 to 7, unlike in Comparative Example 1, the total content of the gadolinium oxide and the cesium oxide was 1% by mass or more. Therefore, while suppressing reduction in radiopacity, the content of the barium oxide was able to be reduced to be less than 30% by mass, and the refractive index was able to be adjusted to be from 1.45 or higher and lower than 1.56.

Although the glass composition according to the present invention has been described above, the present invention is not limited to the above embodiments. Various changes, modifications, substitutions, additions, deletions, and combinations are possible within the scope of the claims recited. These also fall within the technical scope of the present invention.

The present application claims priority to Japanese Patent Application No. 2022-061126, filed with the Japan Patent Office on March 31, 2022, and the entire contents of Japanese Patent Application No. 2022-061126 are incorporated herein by reference.

## Claims

1. A glass composition, comprising:
gadolinium (Gd), cesium (Cs), or both; and
silicon (Si), wherein
in terms of respective oxides thereof, a total content of a gadolinium oxide and a cesium oxide is 1% by mass or more, and a content of a silicon oxide is 30% by mass or more.

2. The glass composition according to claim 1, wherein
the glass composition is in a powder form or in a plate form.

3. The glass composition according to claim 1 or 2, wherein
in terms of the oxide, a content of the gadolinium oxide is 1% by mass or more.

4. The glass composition according to any one of claims 1 to 3, wherein
in terms of the oxide, a content of the cesium oxide is 1% by mass or more.
